## Europäisches Patentamt
## European Patent Office
## Office européen des brevets

(19)

(11) Veröffentlichungsnummer : **0 043 038**
**B1**

(12) # EUROPÄISCHE PATENTSCHRIFT

(45) Veröffentlichungstag der Patentschrift :
22.06.83

(21) Anmeldenummer : 81104697.8

(22) Anmeldetag : 19.06.81

(51) Int. Cl.³ : **C 07 C 57/03**, C 07 C 51/15,
B 01 J 31/24

(54) **Verfahren zur Herstellung von Gemischen von ungesättigten C9-Carbonsäuren.**

(30) Priorität : 01.07.80 DE 3024884

(43) Veröffentlichungstag der Anmeldung :
06.01.82 Patentblatt 82/01

(45) Bekanntmachung des Hinweises auf die Patenterteilung : 22.06.83 Patentblatt 83/25

(84) Benannte Vertragsstaaten :
BE DE FR GB IT NL

(56) Entgegenhaltungen :
DE B 1 520 919
US A 4 167 513
CHEMICAL ABSTRACTS, Band 92, No. 5, 4.
Februar 1980, Columbus, Ohio, USA TAKEHIKO
ITO et al. « Formation of C9 carboxylic acids from
the reaction of carbon dioxide and butadiene in
the presence of palladium complex catalysts and
sodium phenoxide » Seite 724, Spalte 2, Abstract No. 41 273j

CHEMICAL ABSTRACTS, Band 89, No. 23, 4.
Dezember 1978, Columbus, Ohio, USA YOSHIO
INOUE et al. « Incorporation of carbon dioxide in
butadiene dimerization catalysed by palladium
complexes. Formation of 2-ethylidene-5-hepten-
4-olide » Seite 572, Spalte 1, Abstract No. 196
885x

(73) Patentinhaber : EC ERDÖLCHEMIE GMBH
Postfach 75 2002
D-5000 Köln 71 (DE)

(72) Erfinder : Köhler, Hans-Dieter, Dr.
Ostpreussenallee 10
D-4047 Dormagen 1 (DE)
Erfinder : Schleppinghoff, Bernhard, Dr.
Kolpingstrasse 5
D-4047 Dormagen 1 (DE)

(74) Vertreter : Mann, Volker, Dr. et al
c/o Bayer Aktiengesellschaft Zentralbereich Patente
Marken und Lizenzen
D-5090 Leverkusen-Bayerwerk (DE)

## Verfahren zur Herstellung von Gemischen von ungesättigten $C_9$-Carbonsäuren

Die vorliegende Erfindung betrifft ein Verfahren zur Herstellung von Gemischen von ungesättigten $C_9$-Carbonsäuren durch carboxylierende Dimerisation von 1,3-Butadien in Gegenwart eines Palladiumsalzes, eines tertiären Phosphins und Ameisensäure.

Die ungesättigten $C_9$-Carbonsäuren und ihre daraus herstellbaren Ester sind wertvolle organische Zwischenprodukte zur Herstellung von Schmiermitteln, Schmiermittelzusätzen, Weichmachern, Metallseifen, Alkydharzen, Reinigungsmitteln, Emulgatoren, Riech- und Geschmacksstoffen sowie kosmetischen und pharmazeutischen Produkten.

Aus DE-OS 29 05 209 ist es bereits bekannt, ungesättigte $C_9$-Carbonsäureester durch Reaktion von 1, 3-Butadien mit Kohlenmonoxid und einem Alkanol in Gegenwart von Palladiumacetat, Triphenylphosphin und einer tertiären organischen Stickstoffbase bei einem Druck von etwa 50 bar und einer Temperatur von 110 °C in einer 18-stündigen Reaktion herzustellen. Aus US 4 167 513 ist weiterhin bekannt, durch Reaktion von 1,3-Butadien mit Kohlendioxid in Gegenwart von Palladiumacetat und Triphenylphosphin in Tetrahydrofuran als Lösungsmittel bei einem Druck von 50 Atmosphären und einer Temperatur von 50 °C in einer sechsstündigen Reaktion ein Gemisch des 1- und 3-Octadienylester der 2-Ethyliden-hepta-3,5-diencarbonsäure herzustellen.

Aus C.A. *92* (1980), 41 273j ist die Bildung von $C_9$-Carbonsäuren durch Reaktion von $CO_2$ und Butadien in Gegenwart eines Komplex-Katalysators aus Palladiumacetat und Triphenylphosphin bekannt, wobei die Ausbeuten durch Zugabe von Natriumphenolat gesteigert werden soll.

Es wurde nun ein Verfahren zur Herstellung von Gemischen von ungesättigten $C_9$-Carbonsäuren durch Umsetzung von 1,3-Butadien mit $CO_2$ in Gegenwart einer Palladium-(II)-Verbindung und einem tertiären Phosphin gefunden, das dadurch gekennzeichnet ist, daß man die Umsetzung in Gegenwart von Ameisensäure in einem stickstoffhaltigen Lösungsmittel bei 30 bis 150 °C und einem Druck von 4 bis 200 bar durchführt, wobei mindestens 0,5 Mol $CO_2$ pro Mol Butadien eingesetzt werden und dem $CO_2$ gegebenenfalls Wasserstoff beigemischt wird.

Im erfindungsgemäßen Verfahren wird mindestens 1 Mol $CO_2$ pro 2 Mol Butadien, bzw. mindestens 0,5 Mol $CO_2$ pro 1 Mol Butadien eingesetzt. Beispielsweise sei eine Menge von 0,5 bis 5, bevorzugt 0,5 bis 2, besonders bevorzugt 0,5 bis 1 Mol $CO_2$ pro Mol Butadien genannt. Geringere als die genannten Mengen an $CO_2$ erniedrigen in unerwünschter Weise den Umsetzungsgrad und die Ausbeute.

Das erfindungsgemäße Verfahren wird in Gegenwart von nicht-stöchiometrischen Mengen eines Gemisches durchgeführt, das aus einer Palladium-(II)-Verbindung, einem tertiären Phosphin und Ameisensäure besteht. Als Palladium-Verbindung sei beispielsweise das Chlorid, das Bromid, das Nitrat, das Sulfat, das Acetat, das Propionat, das Butyrat oder das Acetylacetonat, bevorzugt das Acetat, genannt. Als tertiäres Phosphin sei beispielsweise das Tripropylphosphin, Triisopropylphosphin, Tributylphosphin, Tricyclohexylphosphin, Trioctylphosphin, Triphenylphosphin, Tri-p-toluylphosphin, Tri-p-methoxyphenylphosphin, bevorzugt das Triphenylphosphin, genannt. Die Mengen der in dem genannten Gemisch anwesenden Stoffe stehen in keinem stöchiometrischen Verhältnis zu den Reaktionspartnern 1, 3-Butadien und $CO_2$. Beispielsweise sei ein Gewichtsverhältnis von Butadien zur Palladium-Verbindung wie 250 bis 2 000 : 1, vorzugsweise 300 bis 350 : 1 und ein Gewichtsverhältnis von Butadien zum tertiären Phosphin wie 100 bis 1 000 : 1, bevorzugt 130 bis 160 : 1, genannt. Die weiterhin in dem genannten Gemisch für das erfindungsgemäße Verfahren enthaltene Ameisensäure wird beispielsweise in einem Molverhältnis Ameisensäure zu Palladium-Verbindung wie 0,1 bis 100 : 1, bevorzugt 0,5 bis 10 : 1, eingesetzt.

In einer bevorzugten Ausführungsform des erfindungsgemäßen Verfahrens wird ein Gemisch aus Palladium-(II)-acetat, Triphenylphosphin und Ameisensäure in den angegebenen Mengen eingesetzt.

Das erfindungsgemäße Verfahren wird in einem stickstoffhaltigen Lösungsmittel in homogener flüssiger Phase in einem Rührkessel oder einem Strömungsrohr durchgeführt. Als stickstoffhaltige Lösungsmittel seien beispielsweise organische Amine, wie Triethylamin, Diethylamin, Pyridin, Tripropylamin, Tributylamin, Triamylamin, Trihexylamin oder sauerstoffhaltige N-Heterocyclen wie N-Methylpyrrolidon, N-Methyl-caprolactam, Pyrrolidon, Piperidon, N-Ethylpiperidon, N-β-Dimethylpropiolactam, N-Ethyl-β-propiolactam genannt. Im erfindungsgemäßen Verfahren werden als Lösungsmittel bevorzugt sauerstoffhaltige N-Heterocyclen, besonders bevorzugt N-Methyl-pyrrolidon (NMP), eingesetzt.

Als Menge des erfindungsgemäß einzusetzenden Lösungsmittels sei beispielsweise ein Gewichtsverhältnis von Lösungsmittel zu Butadien wie 0,5 bis 5 : 1, bevorzugt 1,5 bis 3,5 : 1, genannt.

Es hat sich als vorteilhaft erwiesen und wird daher als besondere Verfahrensvariante betrachtet, daß nach der Herstellung des Gemisches aus der Palladium-Verbindung, dem tertiären Phosphin und der Ameisensäure in dem stickstoffhaltigen Lösungsmittel das Butadien und das $CO_2$ nicht sofort zugegeben werden. Das Butadien und das $CO_2$ werden hingegen erst nach einer kurzen Verweilzeit zu dem vorgelegten beschriebenen Gemisch in dem Lösungsmittel gegeben. Als Verweilzeit sei beispielsweise eine Zeit von 1 bis 60 Minuten, vorzugsweise etwa 5 Minuten, angegeben. Eine Verlängerung der Verweilzeit über 60 Minuten hinaus ist dabei nicht schädlich für das erfindungsgemäße Verfahren. Die Temperatur während dieser Verweilzeit wird bei 0 bis 60 °C, bevorzugt bei etwa 25 °C, gehalten.

Die erfindungsgemäße carboxylierende Dimerisation des 1,3-Butadiens erfolgt bei einer Temperatur von 30 bis 150 °C, vorzugsweise bei 60 bis 110 °C. Der Gesamtdruck des Reaktionssystems liegt beispielsweise im Bereich von 4 bis 200 bar, vorzugsweise im Bereich von 25 bis 90 bar. Der $CO_2$-Partialdruck liegt hierbei im Bereich von 1 bis 150 bar, bevorzugt von 20 bis 80 bar.

Es ist vorteilhaft, das erfindungsgemäße Verfahren unter einer Inertgasatmosphäre durchzuführen. Als inerte Gase seien beispielsweise Stickstoff, Argon oder Methan genannt.

Im erfindungsgemäßen Verfahren können Gemische ungesättigter $C_9$-Carbonsäuren erhalten werden, die beispielsweise 2-Ethyliden-hepta-3,6-diencarbonsäure, 2-Vinyl-hepta-3,6-diencarbonsäure oder 2-Ethyliden-hepta-3,5-diencarbonsäure enthalten.

Es ist weiterhin möglich und als eine besondere Erfindungsvariante anzusehen, simultan zur carboxylierenden Dimerisation eine Einstellung der Anzahl der olefinischen Doppelbindungen zu erreichen. Hierzu wird anstelle von $CO_2$ ein Gemisch aus $CO_2$ und Wasserstoff für die erfindungsgemäße Reaktion verwendet. Der Wasserstoff-Partialdruck wird hierzu im Bereich von 0 bis 40 bar, vorzugsweise 0 bis 10 bar, eingestellt. Dabei wird zunächst bevorzugt die mittelständige Doppelbindung reduziert. Bei steigender $H_2$-Konzentration wird zusätzlich auch die endständige Doppelbindung hydriert, so daß man je nach dem eingestellten $H_2$-Partialdruck ein Gemisch von beispielsweise 2-Ethylidenhepta-6-encarbonsäure und 2-Ethylidenheptancarbonsäure erhält.

Das Gemisch der ungesättigten $C_9$-Carbonsäuren zeigt beispielsweise bei einem Wasserstoff-Partialdruck, der etwa bei 7 bis 12 % des $CO_2$-Partialdruckes liegt, nur noch durchschnittlich 2 olefinische Doppelbindungen pro Molekül und bei einem $H_2$-Partialdruck von etwa 20 bis 30 % des $CO_2$-Partialdruckes nur noch durchschnittlich 1 olefinische Doppelbindung pro Molekül. Diese Ergebnisse können beispielsweise durch die Aufnahme des Massenspektrums nach gaschromatographischer Aufarbeitung des Reaktionsgemisches verfolgt werden.

Beispiele 1 bis 4 (Beispiel 1 ist Vergleichsversuch)

Ein stopfbuchsloser inertisierter 300-m-l $V_4$A-Stahlautoklav mit Magnetrührwerk, Druck- und Temperaturregelung wurde mit einer Katalysatorlösung bestehend aus :

$5,4 \cdot 10^{-4} - 5,4 \cdot 10^{-1}$ Mol HCOOH ; $4 \cdot 10^{-4}$ Mol Palladiumacetat ; $7,78 \cdot 10^{-4}$ Mol Triphenylphosphin ; 10,1 Mol N-Methyl-pyrrolidon (NMP) beschickt.

Nach 5 Minuten wurden bei 25 °C $5,5 \cdot 10^{-1}$ Mol $1,3\text{-}C_4H_6$ zugefügt. Das $CO_2$, gegebenenfalls im Gemisch mit $H_2$, wurde über eine Druckhaltung dem Autoklaven über die Reaktionszeit kontinuierlich zudosiert.

Die Reaktionstemperatur wurde über die Reaktionszeit von 0,5 Stunden in den Grenzen von 75 bis 90 °C geregelt. Der Reaktionsdruck wurde bei den aus der Tabelle ersichtlichen Drücken konstant gehalten.

Die Zusammensetzung des Reaktionsproduktes wurde gaschromatographisch bestimmt, so daß die in der Tabelle angeführten Ausbeuten an $C_9$-Carbonsäuren ermittelt wurden.

Tabelle

| Beispiel | HCOOH (Mol) | $H_2$ (Mol) | $CO_2$ (Mol) | Reaktions-druck (bar) | Ausbeute $C_9$-Carbon-säure (%) |
|---|---|---|---|---|---|
| 1 | $5,4 \cdot 10^{-1}$ | — | $2,76 \cdot 10^{-1}$ | 28 | 17,4 * |
| 2 | $5,4 \cdot 10^{-4}$ | — | $2,76 \cdot 10^{-1}$ | 28 | 24,6 * |
| 3 | $5,4 \cdot 10^{-4}$ | $2,76 \cdot 10^{-2}$ | $2,76 \cdot 10^{-1}$ | 35 | 27,5 ** |
| 4 | $5,4 \cdot 10^{-4}$ | $2,76 \cdot 10^{-2}$ | $2,76 \cdot 10^{-1}$ | 41 | 32,7 ** |

\* Molekulargewicht 152, entspricht einer Summenformel von $C_9H_{12}O_2$ ;
\** Molekulargewicht 154, entspricht einer Summenformel von $C_9H_{14}O_2$ ;

die Werte wurden durch Gaschromatographie und Aufnahme des Massenspektrums erhalten.

**Ansprüche**

1. Verfahren zur Herstellung von Gemischen von ungesättigten $C_9$-Carbonsäuren durch Umsetzung von 1,3-Butadien mit $CO_2$ in Gegenwart einer Palladium-(II)-Verbindung und einem tertiären Phosphin, dadurch gekennzeichnet, daß man die Umsetzung in Gegenwart von Ameisensäure in einem stickstoffhaltigen Lösungsmittel bei 30 bis 150 °C und einem Druck von 4 bis 200 bar durchführt, wobei mindestens 0,5 Mol $CO_2$ pro Mol Butadien eingesetzt werden und dem $CO_2$ gegebenenfalls Wasserstoff beigemischt wird.

2. Verfahren nach Anspruch 1, dadurch gekennzeichnet, daß 250 bis 2 000 Gew.-Teile 1,3-Butadien pro Gew.-Teil Palladium-Verbindung eingesetzt werden.

3. Verfahren nach Anspruch 1, dadurch gekennzeichnet, daß 100 bis 1 000 Gew.-Teile 1,3-Butadien pro Gew.-Teil tertiärem Phosphin eingesetzt werden.

4. Verfahren nach Ansprüchen 1 und 2, dadurch gekennzeichnet, daß 0,1 bis 100 Mol Ameisensäure pro Mol Palladium-Verbindung eingesetzt werden.

5. Verfahren nach Ansprüchen 1 bis 4, dadurch gekennzeichnet, daß das 1,3-Butadien und das $CO_2$ nach einer Verweilzeit von 1 bis 60 Minuten zu dem vorgelegten Gemisch aus Palladium-(II)-Verbindung, tertiärem Phosphin und Ameisensäure in dem stickstoffhaltigen Lösungsmittel gegeben werden.

6. Verfahren nach Anspruch 5, dadurch gekennzeichnet, daß die Temperatur während der Verweilzeit bei 0 bis 60 °C gehalten wird.

7. Verfahren nach Ansprüchen 1 bis 6, dadurch gekennzeichnet, daß anstelle von $CO_2$ ein Mischgas aus $CO_2$ und $H_2$ mit einem Wasserstoff-Partialdruck von 0 bis 40 bar verwendet wird.

**Claims**

1. Process for the preparation of mixtures of unsaturated $C_9$-carboxylic acids by reacting 1,3-butadiene with $CO_2$ in the presence of a palladium-II compound and a tertiary phosphine, characterised in that the reaction is carried out in the presence of formic acid in a nitrogen-containing solvent at 30 to 150 °C and under a pressure of 4 to 200 bars, at least 0,5 mol of $CO_2$ being employed per mol of butadiene and hydrogen optionally being admixed to the $CO_2$.

2. Process according to Claim 1, characterised in that 250 to 2 000 parts by weight of 1,3-butadiene per part by weight of palladium compound are employed.

3. Process according to Claim 1, characterised in that 100 to 1 000 parts by weight of 1,3-butadiene per part by weight of tertiary phosphine are employed.

4. Process according to Claims 1 and 2, characterised in that 0,1 to 100 mols of formic acid per mol of palladium compound are employed.

5. Process according to Claims 1 to 4, characterised in that the 1,3-butadiene and the $CO_2$ are added after a waiting period of 1 to 60 minutes to the mixture, initially introduced, of palladium-II compound, tertiary phosphine and formic acid in the nitrogen-containing solvent.

6. Process according to Claim 5, characterised in that the temperature is kept at 0 to 60 °C during the waiting period.

7. Process according to Claims 1 to 6, characterised in that a mixed gas consisting of $CO_2$ and $H_2$, with a hydrogen partial pressure of 0 to 40 bars is used in place of $CO_2$.

**Revendications**

1. Procédé pour la fabrication de mélanges d'acides carboxyliques en $C_9$ insaturés par réaction de 1, 3-butadiène avec le $CO_2$ en présence d'un composé de palladium (II) et d'une phosphine tertiaire, caractérisé en ce que l'on effectue la réaction en présence d'acide formique dans un solvant azoté à 30-150 °C et sous une pression de 4 à 200 bars, en utilisant au moins 0,5 mole de $CO_2$ par mole de butadiène, le $CO_2$ étant éventuellement mélangé avec de l'hydrogène.

2. Procédé selon la revendication 1, caractérisé en ce que l'on utilise 250 à 2 000 parties en poids de 1,3-butadiène par partie en poids de composé de palladium.

3. Procédé selon la revendication 1, caractérisé en ce que l'on utilise 100 à 1 000 parties en poids de 1,3-butadiène par partie en poids de phosphine tertiaire.

4. Procédé selon les revendications 1 et 2, caractérisé en ce que l'on utilise 0,1 à 100 moles d'acide formique par mole de composé de palladium.

5. Procédé selon les revendications 1 à 4, caractérisé en ce que l'on ajoute le 1,3-butadiène et le $CO_2$ au mélange préalablement chargé de composé de palladium (II), phosphine tertiaire et acide formique dans le solvant azoté après une durée de contact de 1 à 60 min.

6. Procédé selon la revendication 5, caractérisé en ce que la température est maintenue à 0-60 °C pendant la durée de contact.

7. Procédé selon les revendications 1 à 6, caractérisé en ce que l'on utilise au lieu de $CO_2$ un mélange gazeux de $CO_2$ et $H_2$ avec une pression partielle d'hydrogène de 0 à 40 bars.